# EUROPEAN PATENT APPLICATION

(11) **EP 2 966 544 A1**
(43) Date of publication of application: **13.01.2016**
(21) Application number: 14760546.3
(22) Date of filing: 04.02.2014
(51) Int. Cl.: G06F 3/01, H04N 7/08, A61L 9/12

(54) **METHOD AND DEVICE FOR THE EMISSION OF AROMAS/SMELLS IN AUDIOVISUAL PRODUCTS**

(30) Priority: 07.03.2013 ES 201330279
(71) Applicant: Olorama Tecnología, S.L.U., 46900 Torrent (Valencia) (ES)
(72) Inventor: PORCAR RAMÓN, Raúl, E-46900 Torrent (Valencia) (ES)
(74) Representative: Ungria López, Javier
(86) International application number: PCT/ES2014/070076
(87) International publication number: WO 2014/135722

(57) **Abstract**

The procedure for releasing aromas/scents consists of the software playback of an audiovisual product containing at least one coded scent-discharging scene. For each scent-discharging scene in the audiovisual product that is identified and decoded by the software, a coded scent-release signal is sent through wireless communications emitters and received by an aroma/scent release device via wireless communications receivers. As for the aroma/scent release device, it consists of a printed circuit board that commands the synchronized actuation of one or more solenoid valves on an aerosol assembly in order to release aromas/scents, and of one or more fans to facilitate the diffusion of said aromas/scents, based on the coded scent-discharging signal received.

## Description

### TECHNICAL FIELD OF THE INVENTION

This invention applies to the field of displaying audiovisual products, such as films, documentaries, etc., be they shown in movie theaters, playhouses, museums, hotels or in a home setting.

Specifically, we present a procedure and a device that deliver an added value to current methods for displaying audiovisual products by incorporating new sensations via the sense of smell, in addition to those already provided by the senses of sight and hearing, for the enjoyment of said audiovisual products.

### BACKGROUND OF THE INVENTION

The idea of associating aromas/smells with the images and sounds we perceive when watching and enjoying an audiovisual product is not new.

US patent 4629604, published on 16 December 1986, describes a scent player consisting of a container with a series of compartments impregnated with different aromas, each with a heating element connected to it to selectively discharge smells, either manually or synchronized with scenes from an audiovisual product like a video or movie.

The use of this device seems imprecise, slow to activate and difficult to synchronize with the audiovisual product being played.

Likewise, US patent 4905112, published on 27 February 1990, describes a perforated videocassette tape with a compartment where the material with the aroma/smell to be discharged is placed. The air is discharged into the environment by blowing air across the tape as the audiovisual product is played.

This device does not allow for the digital processing of the smell-releasing scenes or their synchronization with the video and audio contents of said audiovisual product.

There is also US patent 6542442, published on 6 December 2001, featuring a smell-discharging device built into a videocassette or other pre-recorded medium and into a device for playing the media contained on said pre-recorded medium. The recording medium features one or more scent-discharging cartridges, and the playback device includes an apparatus for dispersing the scents. The recording also includes inaudible and/or invisible signals that trigger the playback of scents in the emitting device. This playback of scents occurs when the proper signal is received from the recording, which triggers a small pump to release a small amount of the desired scent from the corresponding cartridge. This scent is sprayed into a pressurized chamber, from which it evaporates and is dispersed into the atmosphere by a fan or some other device with this same purpose. Each recording medium can include one or more scent-discharging cartridges, and the playback device includes a series of needles that automatically perforate the cartridges on the recording medium when said medium is inserted into the playback device.

As we can see, the disadvantage of this device is that its use requires a recording medium, be it a videocassette, CD, DVD, etc., as well as a means for playing back the recording, that are especially adapted to trigger the discharge of scents or aromas during playback, as determined by a series of inaudible and/or invisible signals pre-recorded along with the sound and image on the audiovisual products, signals whose actual implementation is unknown.

Thus, a simple and affordable method is needed to modify known technologies for discharging aromas/scents in audiovisual products that can overcome the aforementioned problems, specifically: the need to use complex recording and video playback devices that are adapted for this purpose, as well as the difficulties involved in synchronizing the discharge of aromas/scents with the sounds and images on the audiovisual product.

### DESCRIPTION OF THE INVENTION

This invention is laid out in and characterized by the independent claims, while the dependent claims describe its other characteristics.

In light of the above, this invention involves a procedure for discharging aromas/scents in audiovisual products that entails the following steps:
a) reproduce an audiovisual product with at least one scent-discharging scene coded into the video playback software that can identify and decode said scent-discharging scene,
b) identify and decode the scent-discharging scene in step a),
c) send a coded scent-discharging signal via wireless communications emitters,
d) receive the scent-discharging signal coded in step c) in an aroma/scent discharging device through wireless communications receivers,
e) decode the scent-discharging signal received in step d),
f) convey the scent-discharging signal decoded in step e) to an aroma/scent discharge controller and to an air moving controller,
g) send an activation signal to one or more solenoid valves on an aerosol assembly to release the aromas/scents, based on the scent-discharging signal sent to the aroma/scent discharge controller in step f), and
h) activate one or more fans, depending on the scent-discharging signal sent to the air moving controller in step f).

Also associated with this application is a device for releasing aromas/scents in audiovisual products that is used in steps d) to h) of the procedure described above.

The device features an aerosol assembly containing aroma/scent and a propellant gas. The aerosols are coupled to solenoid valves that control the discharge of the aroma/scent.

Both the aerosols and the solenoid valves are attached to a base structure, with at least one fan placed next to the aerosols to aid in diffusing said aromas/scents into the environment.

The solenoid valves and fans are connected to an aroma/scent discharge controller and to an air moving controller, respectively. Said controllers are located on a printed circuit board, PCB, which commands the synchronized actuation of said solenoid valves and said fans in response to a coded scent-discharge signal received on wireless reception media coupled to the PCB.

As we can see, the device described above improves on and simplifies existing technologies used to discharge aromas/scents during playback of an audiovisual product. This invention manages to efficiently separate the audiovisual playback device from the scent discharging device through the wireless operation of the latter, which can be placed in the most suitable location, independently of the position of the playback device, be it in a public setting (theater, playhouse, museum, hotel, etc.) or in a home setting.

Since the scent-discharging scenes are coded alongside the audio and sound on the audiovisual product, said scenes being efficiently identified and decoded by software on the video playback device, an unlimited diversity of aromas/scents can be released, either singly or jointly, as desired, in synchronization with the sound and image, thus providing greater realism to the resulting sensations in viewers of the audiovisual product.

The audiovisual product containing the encoded scent-discharging scenes can be designed for this purpose from the start or converted after the fact (either through analog or digital technology) by coding said scenes into the digital version of the product prior to reproduction in a video playback device that is capable of identifying and decoding said scenes. Likewise, the scent-discharging scenes coded into the audiovisual product will not hamper the product's playback on a video reproduction device that is not capable of identifying and decoding said scent-discharging scenes, though its viewers will not enjoy the additional sensations provided by the discharge of aromas/scents during the play back of audiovisual products.

### BRIEF DESCRIPTION OF THE FIGURES

For a better understanding of this invention, we include a series of figures representative of the preferred, though not limiting, embodiment of the invention.
Figure 1 shows a schematic top view of the device for discharging aromas/scents in audiovisual products.
Figure 2 shows a schematic side view of one of the aerosols and its associated solenoid valve of the device in Figure 1.
Figure 3 shows a schematic front view of a preliminary design of the base structure for the aerosols and solenoid valves of the device in Figure 1.
Figure 4 shows a top perspective of the base structure containing the aerosols and solenoid valves shown in Figure 3.
Figure 5 shows a top perspective of a secondary design of the base structure for the aerosols and solenoid valves of the device in Figure 1.
Figure 6 shows a schematic for connecting the electronic components on the printed circuit board and the solenoid valves and fans on the device in Figure 1.

### DETAILED PRESENTATION OF THE PREFERRED EMBODIMENT OF THE INVENTION

The procedure for discharging aromas/scents in audiovisual products in this invention involves playing an audiovisual product with at least one scent-discharging scene coded into the video playback software that can identify and decode said scent-discharging scene.

As we can see, before the audiovisual product is played on the video playback software, at least one scent-discharging scene must be coded into the audiovisual product. To this end, the digitalized audiovisual product should be played on video playback software that can code scent-discharging scenes contained in the audiovisual product such that, once an image from a scene that is to be coded to discharge a scent, the playback of the audiovisual product is halted and the scent-discharging scene is coded into said image.

The playback of the audiovisual product, the stopping of playback at the image of interest and the coding of the scent-discharging scene at said image of interest can be repeated for as often as there are images of interest for coding scent-discharging scenes into the audiovisual product.

The audiovisual product, with at least one coded scent-discharging scene, is then saved. The video editing software stores the information on the scent-discharging scenes within the video file itself. This file can be called an SDA (Scent Description Archive) and can be based on the XML (extended Markup Language) standard.

For greater security, the SDA should be stored encrypted using a dynamic key system, thus achieving greater protection by allowing cracked keys to be invalidated.

The video-editing software should also be designed under a service-based architecture such that one-time changes can be made to the video without requiring a complete reprogramming. Specifically, we foresee these changes being made in one of three modules:
- in the module where the contents of the SDA file are created,
- in the module implementing the code and that encapsulates the scent content of each scene in the video file, and
- in the module where the video file is encrypted.

Given this example of the preferred embodiment of how to code at least one scent-discharging scene in the audiovisual product, we now return to the playback of said audiovisual product in the video playback software. During said playback, the scent-discharging scene is identified and decoded. This sends a coded scent-release signal through wireless communications emitters.

Preferably, the coded scent-discharging scene and the coded scent-release signal will transmit information about the type(s) of aroma(s)/scent(s) to be released, as well as about the time and duration of the release.

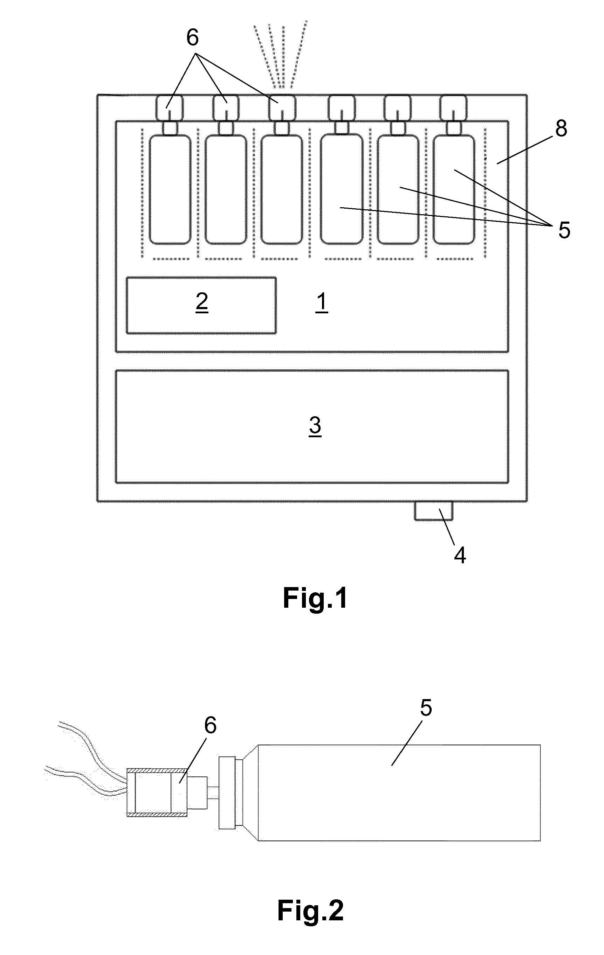

Subsequently, said coded scent-release signal is received in an aroma/scent releasing device, as seen in Figures 1 through 6, through wireless communications receivers (2). Said scent-releasing signal is decoded and sent to an aroma/scent discharge controller (10) and to an air moving controller (9).

An activation signal is then sent to one or more solenoid valves (6) in an aerosol assembly (5) to release the aromas/scents, based on the scent-release signal sent to the aroma/scent discharge controller (10). At the same time, one or more fans (7) is placed in operation, based on the scent-release signal sent to the air moving controller (9).

Preferably, during playback of the audiovisual product in the video playback software, the corresponding coded scent-release signal should be sent while the scent-discharging scene is identified and decoded. Said signal will be received in the aroma/scent discharging device, as seen in Figures 1 to 6, and decoded before being sent to the aroma/scent discharge controller (10) and to the air moving controller (9), which send activation signals both to the solenoid valves (6) and to the fans (7), respectively, so that these can be turned on based on the information contained in the coded scent scene included in the audiovisual product.

Likewise, the video playback software which:
- plays the audiovisual product with at least one coded scent-discharging scene,
- identifies and decodes said scent-discharging scene, and
- sends a coded scent-release signal based on the scent-discharge scene,
should preferably run on a PC or an electronic device such as a tablet, smartphone, Smart TV, etc.

As for the aroma/scent discharge device that receives the coded scent-release signal through wireless communications receivers (2), such as via Wi-Fi, it features an aerosol assembly (5) that contains aromas/scents and a propellant gas.

As seen in Figure 2, the aerosols (5) are coupled to solenoid valves (6) that control the discharge of the aroma/scent.

As Figures 1, 3, 4 and 5 show, both the aerosols (5) and the solenoid valves (6) are attached to a base structure (8), with at least one fan (7) placed next to the aerosols (5) to aid in diffusing said aromas/scents into the environment.

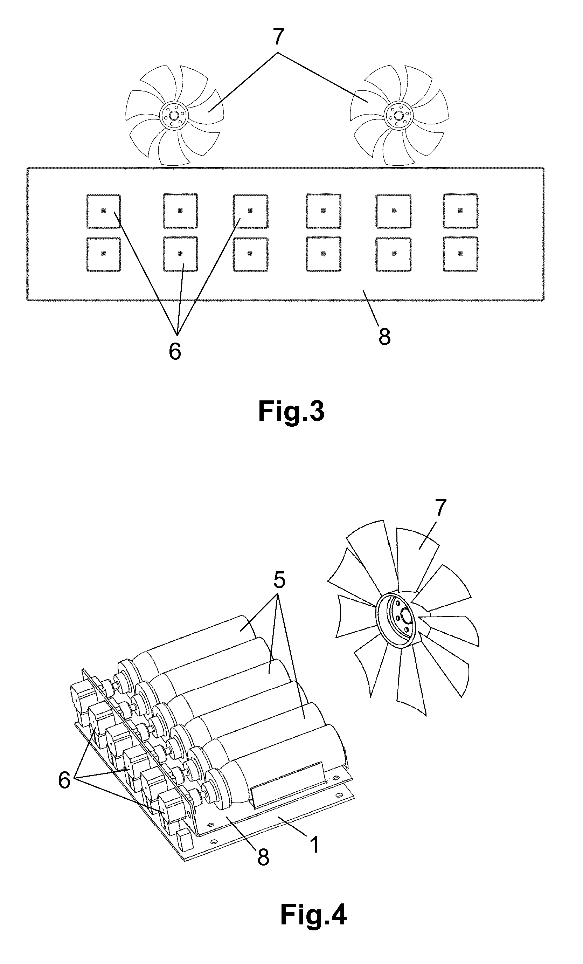

In the first example of the embodiment, shown in Figures 3 and 4, the base structure (8) is flat, with the aerosols (5) and their associated solenoid valves (6) arranged in a straight line atop the base structure (8), that is, equidistantly separated from one another at the same height. In this case, the fans (7) will preferably be laid out atop the base structure (8).

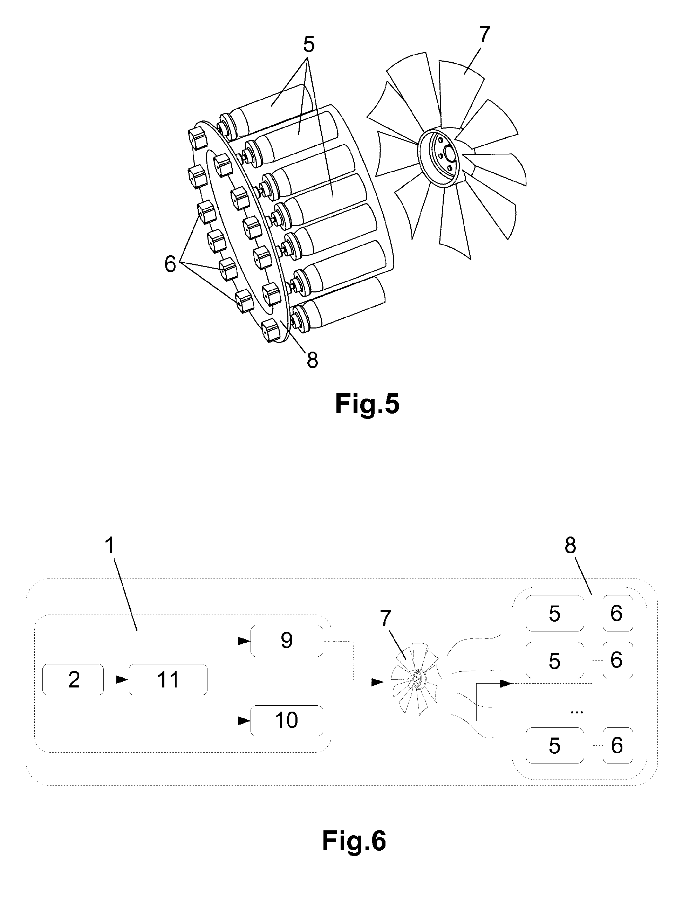

Figure 5 shows a second example of the embodiment where the base structure (8) is laid out in a "cannon" shape, that is, cylindrically, with the aerosols (5) and their associated solenoid valves (6) arranged in a circular line on the outside of said base structure (8). In this case, the fan (7) can be behind the base structure (8).

As Figure 6 shows, the solenoid valves (6) and fans (7) are connected to an aroma/scent discharge controller (9) and to an air moving controller (10), respectively.

The controllers (9, 10) are located on a printed circuit board (1), PCB, that commands the synchronized actuation of said solenoid valves (6) and said fans (7) in response to a coded scent-discharge signal received on wireless communications receivers (2) coupled to the PCB (1).

As for the printed circuit board (1), it should contain a signal decoder (11) that decodes the scent-release signal received on the wireless communications receivers (2) and sends it simultaneously to the aroma/scent discharge controller (9) and to the air moving controller (10).

Likewise, the device should have at least one battery (3) to supply electricity to the printed circuit board (1) and to the fans (7) for the self-sufficient operation of the device. The battery (3) features an external connector (4) that can be plugged into a wall outlet (not shown in the figures) to be recharged. Reduced-energy components are used to increase the device's battery life.

As we can see, both the flexibility in supplying the printed circuit board (1) and the fans (7) (via rechargeable batteries) and in wirelessly (without cables) communicating between the video playback software and the aroma/scent discharging device allow the device to be installed in the most suitable place in any type of setting, whether public (theaters, playhouses, museums, hotels, etc.) or in the home.

## Claims

1. Procedure for releasing aromas/scents in audiovisual products consisting of the following steps:
a) reproduce an audiovisual product with at least one scent-discharging scene coded into the video playback software that can identify and decode said scent-discharging scene,
b) identify and decode the scent-discharging scene in step a),
c) send a coded scent-discharging signal via wireless communications emitters,
d) receive the scent-discharging signal coded in step c) in an aroma/scent discharging device through wireless communications receivers,
e) decode the scent-discharging signal received in step d),
f) convey the scent-discharging signal decoded in step e) to an aroma/scent discharge controller and to an air moving controller,
g) send an activation signal to one or more solenoid valves on an aerosol assembly to release the aromas/scents, based on the scent-discharging signal sent to the aroma/scent discharge controller in step f), and
h) activate one or more fans, depending on the scent-discharging signal sent to the air moving controller in step f).

2. Procedure as per claim 1, in which, prior to step a), at least one scent-discharging scene has been coded into the audiovisual product in keeping with the following steps:
i) play back the audiovisual product on video editing software that is capable of encoding scent-discharging scenes in said audiovisual product,
ii) stop playback of the audiovisual product at an image of interest to encode a scent-discharging scene,
iii) encode the scent-discharging scene in the image in step ii),
iv) save the audiovisual product with at least one scent-discharging scene coded in step iii).

3. Procedure as per claim 2, in which steps i) to iii) are repeated for as long as there are images associated with scent-discharging scenes to be encoded in the audiovisual product.

4. Procedure as per claim 1, in which steps b) to h) are repeated for as long as scent-discharging scenes are identified and decoded in the audiovisual product played back in step a).

5. Procedure as per claim 1, in which, by means of the coded scent-discharging scene and the coded scent-release signal, information is transmitted about the type(s) of aroma (s)/scent (s) to be released, as well as about the time and duration of the release.

6. Procedure as per claim 1, in which the video playback software that carries out steps a) to c) is executed on a PC or on an electronic device such as a tablet, smartphone or Smart TV.

7. Device for releasing aromas/scents in audiovisual products, used in steps d) to h) of the procedure in claim 1, which comprises an aerosol assembly (5) containing aromas/scents and a propellant gas, the aerosols (5) being coupled to associated solenoid valves (6) that control the release of the aromas/scents, wherein both the aerosols (5) and the solenoid valves (6) are attached to a base structure (8), and there is at least one fan (7) located next to the aerosols (5) to facilitate the diffusion of said aromas/scents into the atmosphere, **characterized in that** the solenoid valves (6) and the fans (7) are connected to an aroma/scent discharge controller (9) and to an air moving controller (10), respectively, wherein said controllers (9 and 10) are part of a printed circuit board (1) that commands the synchronized actuation of said solenoid valves (6) and said fans (7) based on a coded scent-release signal received via wireless communications receivers (2) coupled to the printed circuit board (1).

8. Device as per claim 7, in which the printed circuit board (1) contains a signal decoder (11) that decodes the scent-release signal received on the wireless communications receivers (2) and sends it simultaneously to the aroma/scent discharge controller (9) and to the air moving controller (10).

9. Device as per claim 7, wherein the electrovalves (6) are solenoid electrovalves.

10. Device as per claim 7, in which the base structure (8) is flat with the aerosols (5) and their associated solenoid valves (6) arranged in a straight line atop said base structure (8).

11. Device as per claim 10, in which the fans (7) are laid out atop the base structure (8).

12. Device as per claim 7, in which the base structure (8) is arranged in a "cannon shape" with the aerosols (5) and their associated solenoid valves (6) laid out in a circular line on the outside of said base structure (8).

13. Device as per claim 12, in which the fan (7) is laid out behind the base structure (8).

14. Device as per claim 7, which contains at least one battery (3) to supply electricity to the printed circuit board (1) and to the fans (7) for the self-sufficient operation of the device.
